# EUROPEAN PATENT APPLICATION

(11) **EP 1 932 488 A1**
(43) Date of publication of application: **18.06.2008**
(21) Application number: 07022507.3
(22) Date of filing: 20.11.2007
(51) Int. Cl.: A61B 19/00, B25J 9/16

(54) **Frame of reference registration system and method**

(30) Priority: 12.12.2006 GB 0624770
(71) Applicant: Prosurgics Limited, High Wycombe Buckinghamshire HP10 9QR (GB)
(72) Inventor: Finlay, Patrick, High Wycombe Bucks HP10 9QR (GB); Gullam, Richard, High Wycombe Bucks HP10 9QR (GB)
(74) Representative: Beattie, Alex Thomas Stewart

(57) **Abstract**

A system for assisting in work carried out on a workpiece and having a frame of reference, the system comprising: a referencing arrangement to register the position of a first location in the frame of reference of the system; a tool holder for holding a tool to assist with the work, the position of the tool holder being known within the frame of reference of the system; a data interface to receive image data relating to the workpiece, the image data representing an image which is indexed by position relative to the first location; and a processing arrangement to register the image data within the frame of reference of the system by utilising the relative position of the image represented by the image data with respect to the first location and the position of the first location in the frame of reference of the system.

## Description

### Description of Invention

The present invention relates to a method of registration of a workpiece within a frame of reference. In particular, the present invention relates to the registration of the location of a workpiece within the frame of reference of a robot or other device utilising one or more previously acquired images of the workpiece.

When a workpiece is to be acted upon it is sometimes necessary to register the actual location of the workpiece with images thereof to ensure that any work is carried out on a correct region of the workpiece. For example, an image of the internal structure of the workpiece may be acquired and used as a guide when work is carried out on part of the internal structure of the workpiece which is not externally visible.

In such instances, the frame of reference used to acquire the images of the workpiece must be matched with the frame of reference in which subsequent work is carried out such that it is possible to direct a tool on or in the workpiece to act upon an area of interest (such as part of the internal structure of the workpiece). The tool may be directed utilising images of the workpiece which are acquired earlier; however, directing a tool in this manner is difficult because the actual orientation of the workpiece is usually different to the orientation of the workpiece when the earlier images were acquired. In addition, the format of the images may not be conducive to such work. For example, image slices of a workpiece may depict the workpiece in its actual orientation but directing a tool based upon image slices may not be an easy procedure.

Generally, in order to register images of a workpiece with the subsequent location of the workpiece it is necessary to utilise features of the workpiece which are visible in both the image and in a view of the actual workpiece.

Alternatively, fiducial markers may be attached to the workpiece such that they are visible on the external surface thereof. The location of these markers within the images can be registered with the actual location of the markers on the workpiece and, thus, the location and orientation of the workpiece can be determined and matched with the images.

For example, such techniques are utilised in surgical operations during which images are initially acquired using an MRI or CT scanner (or other imaging device/modality) to record the internal structure of part of a patient. Fiducial markers are adhered to the patient's skin or embedded in one of the patient's bones. These fiducial markers are visible in the MR or x-ray CT images which are obtained during the scanning process. Subsequently, a surgical operation is carried out on the patient utilising the MRI or x-ray images.

The use of fiducial markers and similar techniques introduces a number of problems. For example, if the fiducial markers become displaced, then the actual location of the workpiece (or patient in the example provided above) cannot be accurately registered within the frame of reference of the robot or matched with the images of the workpiece which were captured earlier.

In addition, the processing techniques required to register the workpiece within the frame of reference to the robot are complex and often take a considerable amount of time to complete.

The present invention seeks to ameliorate the problems associated with the

### prior art.

In order that the present invention may be more readily understood, embodiments thereof will be described, by way of example, with reference to the accompanying drawings, in which:
FIGURE 1 shows a CT scanner; and
FIGURE 2 shows a table for use with an image acquisition device such as a CT or MRI scanner; and
FIGURE 3 shows a robot according to an embodiment of the present invention.

The present invention shall now be described by way of reference to a surgical operation in which the workpiece is a patient. However, it will be appreciated that the present invention is equally applicable to use in relation to other workpieces and other procedures. For example, embodiments of the present invention could be utilised within a manufacturing facility or as part of a automated production line.

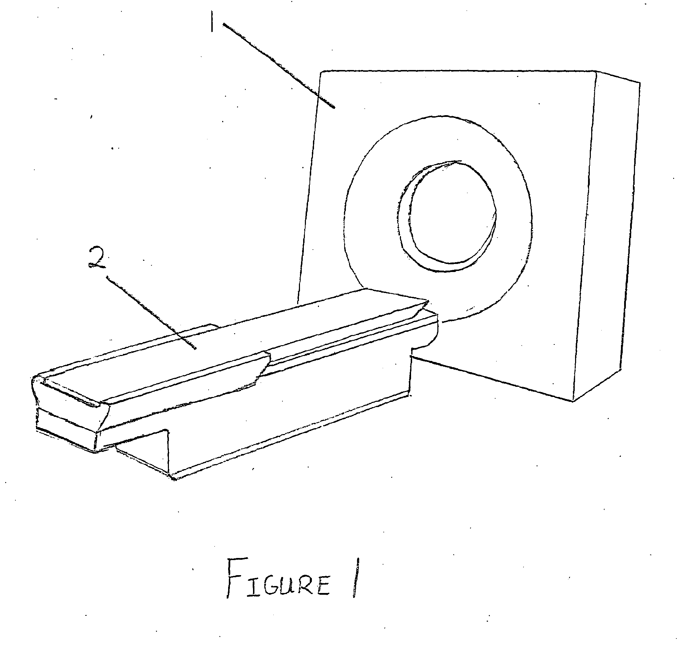

Prior to a surgical operation a patient may be scanned to obtain internal images of an area of the patient's body. For example, images of a patient's spine may be acquired prior to an operation to correct a deformity or to treat a trauma. An example of a CT scanner 1,2 is shown in Figure 1.

It will be appreciated that the type of imaging device utilised to obtain images of the patient will be dependent upon a number of factors. These factors include the availability of the imaging devices, the type of information required, the characteristics of the patient, and the cost associated with the use of the device.

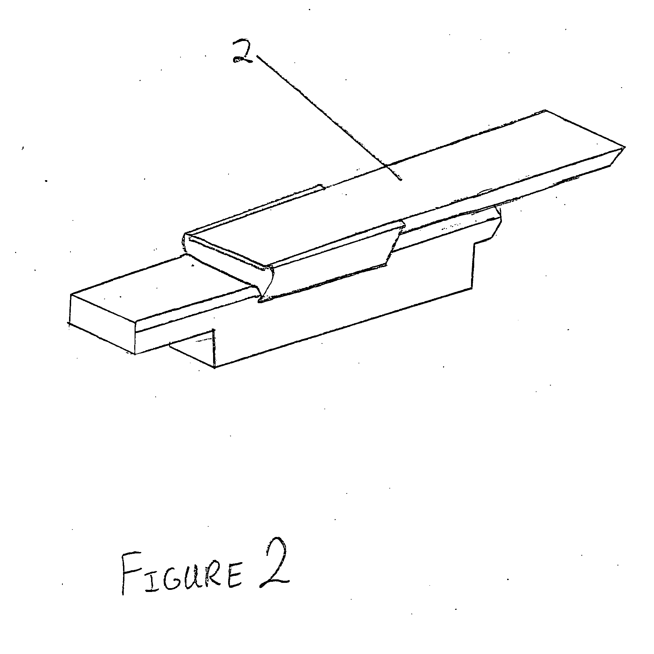

Many imaging devices acquire images using standard file formats in which image slices of a patient (usually sagittal, coronal or axial) are directly related to the position of the bed 2 (or "table") - on which the patient is placed - within the device when the images are acquired (ie. the image data is indexed with respect to the relative position of the table 2). Figure 2 shows a typical table 2 in a second position (Figure 1 which includes a view of a CT scanner 1 shows the table 2 in a first position)

In some devices each image voxel may be associated with a coordinate value representing its location in three dimensional space relative to a location on the table 2. This includes examples of devices in which each image slice is associated with a value representing the position of the table 2 with respect to the imaging device when the slice was acquired. In this instance, each voxel is associated with three dimensional coordinates by virtue of its position within the particular image slice (ie. its two dimensional position within the image) and the value associated with the slice.

An example of a file format which is utilised by many imaging devices is the Dicom file format and, more specifically, version three of that file format.

This file format has been widely introduced so that images obtained using different imaging devices (which need not be different types of imaging device) can be processed and manipulated by many different devices, including peripheral equipment.

Example imaging techniques or modalities which are supported by the Dicom file format include: computed topography (CT), magnetic resonance (MR), ultrasound and computed radiography (CR). It will be appreciated that there are a vast number of additional modalities which are supported by file formats such as the Dicom file format. The present invention is not limited by the specific use of the Dicom file format which is merely utilised as an example of a suitable file format. Nor is the present invention limited to the use of a particular modality or imaging device.

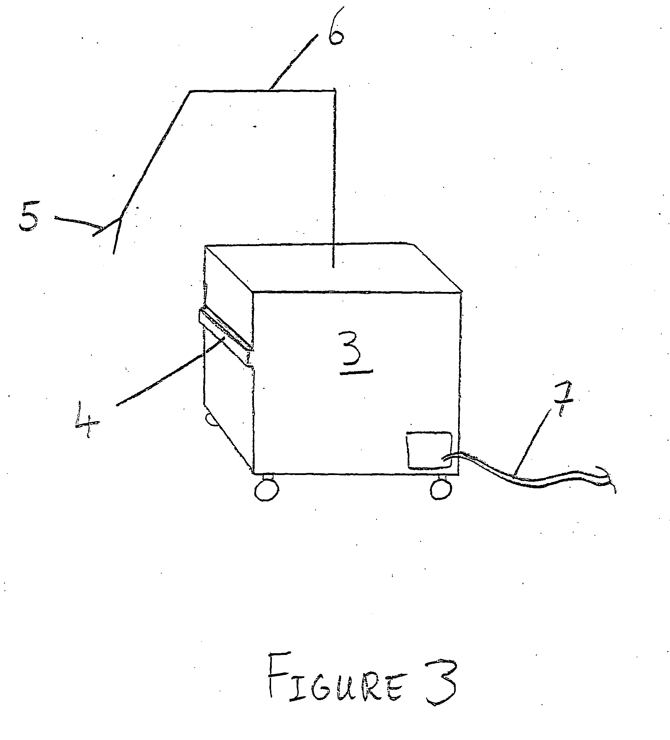

If a tool holder, robot, tool or other device 3 is placed in a position relative to the table 2 on- which the patient was placed during the preparatory image acquisition process (as discussed above and shown in Figure 3), then the table position can be determined relative to the robot 3 (or other device); this information can be utilised to register the location of the patient within the frame of reference of the robot by matching the current table position with table position in at least one of the image slices which were acquired during the image acquisition process - the images being referenced with respect to a known location on the table 2.

Using this technique, each voxel within a three-dimensional image (comprising, for example, a number of image slices) of the patient which was acquired during the image acquisition process may be registered to a physical three-dimensional co-ordinate within the frame of reference of the robot 3. Thus, it is possible for the robot 3 to carry out delicate work on the patient with a reduced risk of error.

In order to minimise the problems associated with the patient moving relative to the table 2 after the image acquisition process but before the surgical operation (or "intervention"), it is preferable to carry out any surgical procedure within the vicinity of the imaging device; for example, within the scanner suite in which the images are acquired.

The placement of a robot 3 in a location relative to the position of the table 2 utilised in the image acquisition process such that the position of the table 2 may be registered within the frame of reference of the robot 3 can be achieved in a number of different manners. For example, the robot 3 can be permanently attached to the table 2 at a known location. In such an instance the robot 3 may also be subjected to the image acquisition process. It will be appreciated that it may be difficult to utilise such a robot 3 in conjunction with certain imaging devices - for example, in a MRI strong magnetic fields could make the use of a permanently attached robot 3 difficult.

Furthermore, in a number of imaging devices the table on which the patient is placed must be passed through a bore in the scanning device. This places severe restrictions upon the dimensions of any robot 3 which is permanently attached to the table. Furthermore, a robot 3 of this type may cause an obstruction to the imaging device.

Alternatively, it is possible to attach the robot 3 temporarily to the table after the scanning process. This can be achieved by providing an attachment arrangement (not shown) on the table 2 at a known location and a corresponding attachment arrangement 4 on part of a robot 3.

Preferably, the robot 3 is separate from the scanner table 2 and moved into a position generally adjacent to the table 2. The robot 3 may be freestanding and self-contained (with the possible exception of a power supply). In this instance, the robot 3 includes a referencing arrangement 4 such that it is possible to register the position of the table 2 within the frame of reference of the robot 3 by utilising the referencing arrangement 4.

The referencing arrangement 4 could take a number of forms. For example, the arrangement 4 may include one or more location registering elements which can be abutted against one or more corresponding locating elements on the table. The one or more location registering elements of the robot 3 may have a fixed location with respect to the location of the robot 3 or may moveable with respect to the location of the robot (or a combination of both).

In the latter case the position of the location registering elements of the robot 3 can, according to one aspect of the invention, be determined using magnetically encoded tape along a surface which is fixed with respect to the location of the robot 3. Movement of a location registering element, in such an arrangement, would cause a corresponding movement in a magnetic information reading device (or decoder) suitable to read the encoded tape such that-the location of the element can be determined with respect to the location of the robot. It will be appreciated that additional referencing arrangements 4 may be needed if the location registering elements can move in more than one axis.

Other referencing arrangements 4 include the use of laser interferometry, triangulation techniques, stereo images (captured by, for example, one camera moved to multiple locations or by two or more cameras), and contact or noncontact trigger probes (or other metrology techniques). In some instances a tool 5 attached to an arm 6 of the robot 3 is manoeuvred into a position such that the tool 5 is in contact with a known location on the table 2. Alternatively, at least part of the robot 3 may be inserted into the imaging device and the resultant image of the part of the robot 3 can be used for referencing, combined with knowledge of its own position from its joint encoders.

In other embodiments of the present invention the robot 3 has a referencing arrangement 4 comprising one or more fixed receptacles or surfaces into which or against which at least part of the table 2 can be placed. It will be appreciated that such surfaces or receptacles could be used to register the location of the table 2 within the frame of reference of the robot 3.

It will be understood that the method used to register the position of the table 2 in the frame of reference of the robot 3 can take a number of forms. The arrangements provided above are merely examples of such methods.

The referencing can occur while the patient and table 2 are still within or close to the imaging device. The table 2 can then be moved out of the imaging device to allow more access to the patient. The movement of the table 2 can be recorded, for example, by the imaging device and this information passed to the robot 3. Therefore, a table 2 which has been registered within the frame of reference of the robot 3 in a first position may be subsequently moved to a second position and the- movement recorded. The robot 3 will be able to adjust the position of the table 2 within its frame of reference without the need to re-register the location of the table 2 by using the recorded movement information.

Once the location of the table 2 has been registered within the frame of reference of the robot 3, then previously acquired image information can be matched with the known location of the table 2 to register the location of the patient (ie. the workpiece) within the frame of reference of the robot 3. This information is acquired though a data interface 7.

The data interface 7 may be directly linked to an imaging device or may comprise a connection to a network (such as an Ethernet connection). The interface may be wired or wireless.

The robot 3 uses the coordinate information associated with the voxels in the image information to register the location of the patient within its frame of reference. In other words, the robot 3 uses the known location of the table 2 with information which relates to the position of the patient on the table 2, in order to determine the actual location of the patient.

A robot 3 according to an embodiment of the present invention may, therefore, comprise: one or more referencing arrangements 4 (to determine the location of the table with its frame of reference), a data interface 7 (to receive information concerning the images acquired during the image acquisition process and information concerning any movements of the table), and a processing arrangement (not shown) suitable to register the location of the table 2 within the frame of reference of the robot 3 and match the image information with the frame of reference. Preferably, the processing arrangement allocates one or more three-dimensional coordinate values within the frame of reference of the robot 3 (ie. potentially different coordinate values to those associated with the voxel and stored in the image information) to one or more respective voxels of the images.

The robot 3 advantageously includes one or more tools 5, or tool attachment arrangements (not shown) to accept tools 5 - the tools 5 being, for example, suitable to act on the patient. After the patient has been registered within the frame of reference of the robot 3, the robot 3 can operate to perform a task in relation to the patient.

The term "robot" has been used above; however; this term is intended to include fully and semi-automated devices capable of controlling, assisting or actually working on a workpiece (eg. a patient). The system need not, however, include a robot, and may for example alternatively include a passive tool holder. A tool held by the tool holder may itself comprise a surgical robot.

In some embodiments of the present invention the robot 3 is also operable to locate one or more features of the workpiece or one or more fiducial markers (not shown) attached to or placed on the workpiece to aid- in the registration process.

It will be appreciated that the table 2 is only an example of a first type of object relative to which a workpiece (or second type of object) may be located. A workpiece can be placed relative to any known location (to form the basis of the coordinate values of the image information) so long as the robot 3 can determine the location of a point which has a known position (by virtue of, for example, a coordinate value) with respect to the known location. In other words the robot 3 must be able to determine the basis on which the coordinates associated with the image voxels has been made (this usually requires information about the location of the origin of the coordinate system and the spacing of coordinate values).

When used in this Specification and Claims, the terms "comprises" and "comprising" and variations thereof mean that the specified features, steps or integers are included. The terms are not to be interpreted to exclude the presence of other features, steps or components.

The features disclosed in the foregoing description, or the following Claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A system for assisting in work carried out on a workpiece and having a frame of reference, the system comprising:
a referencing arrangement to register the position of a first location in the frame of reference of the system;
a tool holder for holding a tool to assist with the work, the position of the tool holder being known within the frame of reference of the system;
a data interface to receive image data relating to the workpiece, the image data representing an image which is indexed by position relative to the first location; and
a processing arrangement to register the image data within the frame of reference of the system by utilising the relative position of the image represented by the image data with respect to the first location and the position of the first location in the frame of reference of the system.

2. A system according to claim 1, wherein the tool holder comprises a robot.

3. A system according to claim 2, wherein the robot is a medical robot.

4. A system according to any preceding claim, wherein the referencing arrangement comprises a laser interferometer.

5. A system according to any one of claims 1 to 4, wherein the referencing arrangement comprises a triangulation device.

6. A system according to any one of claims 1 to 4, wherein the referencing arrangement comprises one or more surfaces for abutment against part of an object associated with the first location.

7. A system according to any preceding claim, wherein data interface is operable to receive image data in the Dicom 3 file format.

8. A system according to any preceding claim, wherein the data interface is operable to receive image data in the form of image slices, each image slice being indexed by position with respect to the first location.

9. A system according to any preceding claim, further comprising a referencing adjustment arrangement operable to adjust the position of the first location within the frame of reference of the system as a result of information received by the system concerning a movement of the first location.

10. A method of registering image data within a frame of reference of a system including a tool holder for holding a tool for assisting in work carried out on a workpiece, the position of the tool holder being known within the frame of reference of the system, the method comprising:
registering the position of a first location in the frame of reference of the system;
receiving image data relating to the workpiece, the image data representing an image which is indexed by position relative to the first location; and
registering the image data within the frame of reference of the system by utilising the relative position of the image represented by the image data with respect to the first location and the position of the first location in the frame of reference of the system.

11. A method according to claim 10, wherein the step of providing a tool holder comprises the step of providing a robot.

12. A method according to claim 11, further comprising the step of providing a medical robot as the robot.

13. A method according to any one of claims 10 to 12, further comprising the step of providing a laser interferometer to register the position of the first location.

14. A method according to any one of claims 10 to 12, further comprising the step of providing a triangulation device to register the position of the first location.

15. A method according to any one of claims 10 to 12, further comprising the step of providing one or more surfaces for abutment against part of an object associated with the first location to register the position of the first location.

16. A method according to any one of claims 10 to 15, further comprising the step of providing image data in the Dicom 3 file format.

17. A method according to any one of claims 10 to 16, wherein the step of receiving image data comprises the step of receiving image data in the form of image slices, each image slice being indexed by position with respect to the first location.

18. A method according to any one of claims 10 to 17, further comprising the step of acquiring image data using an image acquisition device.

19. A method according to claim 18, wherein the step of acquiring image data comprises the step of acquiring image data including the tool holder.

20. A method according to any one of claims 10 to 19, further comprising the step of adjusting the position of the first location within the frame of reference of the system as a result of information received by the system concerning a movement of the first location.

21. A computer program operable to control a system to perform the method of any one of claims 10 to 20.

22. A computer program according to Claim 21, embodied on a computer readable medium
